(19) Europäisches Patentamt
European Patent Office
Office européen des brevets

(11) Publication number: **0 330 358 A2**

# (12) EUROPEAN PATENT APPLICATION

(21) Application number: 89301357.3

(22) Date of filing: 13.02.89

(51) Int. Cl.4: C12Q 1/38 , G01N 33/564 , A61K 35/00

(30) Priority: 11.02.88 GB 8803109

(43) Date of publication of application: 30.08.89 Bulletin 89/35

(84) Designated Contracting States: AT BE CH DE ES FR GB GR IT LI LU NL SE

(71) Applicant: SERVASE LIMITED
Chalfont House 35 Churchfield Road
Acton London W3 6AY(GB)

(72) Inventor: Worman, Colin Peter
20 Adderley Road
Harrow Weald Middlesex HA3 7HT(GB)
Inventor: Habib, Nagy Adly
8 Nichols Green
Ealing London W5 2QU(GB)
Inventor: Apostolov, Kosta
15 Canterbury Close
Beckenham Kent BR3 2EP(GB)
Inventor: Barker, William Roland
3 Braintree Road
South Ruislip Middlesex(GB)
Inventor: Wood, Christopher Barry
Gamekeepers Cottage Pinewood Road
Iver Heath Buckinghamshire SL0 0NJ(GB)

(74) Representative: Abrams, Michael John et al
HASELTINE LAKE & CO. Hazlitt House 28
Southampton Buildings Chancery Lane
London WC2A 1AT(GB)

## (54) Improvements in or relating to clinical testing, monitoring, and pharmaceutical compositions for treatment of auto-immune diseases.

(57) The involvement of serine esterase activity in a number of clinical conditions is disclosed. A rapid test for serine esterase activity is disclosed, this test involving (a) fixing the sample which is to be tested, e.g. in formalin; and (b) contacting the fixed sample with a solution containing N-alpha benzyloxy carbonyl-L-lysine thiobenzyl ester and a chromogen (e.g. Fast Blue BB). The use of this test as a diagnostic indicator or monitor of the progress of therapeutic treatment is disclosed. Techniques for the treatment of diseases, particularly auto-immune diseases, are also suggested.

EP 0 330 358 A2

# IMPROVEMENTS IN OR RELATING TO CLINICAL TESTING, MONITORING, AND PHARMACEUTICAL COMPOSITIONS FOR TREATMENT OF AUTO-IMMUNE DISEASES

This invention relates to the detection of clinically significant moieties, to clinical testing and monitoring, and to pharmaceutical compositions for the treatment principally (but not exclusively)of auto-immune diseases. In one aspect, the invention is particularly concerned with a method for determining the localisation and distribution of the cytotoxicity-associated serine esterase in haematological cells.

In recent years, the presence of a serine esterase (SE) or putatative protease in lysates of malginant and transformed cytotoxic T cell lines, cloned cytotoxic T-cell lines and ex-vivo cell preparations has been described. This serine esterase activity has also been found in some cell lines with a phenotype usually indicative of a helper function, but which also have the capacity to kill specific antigen bearing target cells.

The connection between SE and cell cytolytic potential was further exemplified by the co-appearance and co-inducibility of messenger RNA transcripts CTLA-1, HF, B10 and C11, all sharing some homology with other mammalian SE and the cytotoxic capacity of lymphocytes in both murine and the human systems.

There is now considerable evidence that cellular proteases play an important role in cell mediated cytotoxicity. The interaction between a cytotoxic T-lymphocyte (CTL) and its target cell results in the specific lysis of the latter. Sagury (1982) was the first to propose that a lethal hit of a target cell by an effector cell was the result of an exocytosis of cytolytic molecules from the CTL.

Subsequent work has shown that there are several components contained in the large electron-dense cytoplasmic granules in cloned CTLs and natural killer (NK) cells which are thought to be involved in the killing process. One such component, a protein called Perforin (Podack et al 1983) has been studied by a number of workers. It has a molecular weight of 70-75,000 under non-reducing conditions and 62-65,000 under reducing conditions (Young et al 1986) and exhibits functional and structural similarities to the C9 component of the humoral complement system (Tschopp et al 1986, Young et al 1986 and Zalman et al 1986). Perforin is secreted into the intercellular space from CTLs and NK cells by a process of degranulation following effector-target cell interaction, and in the presence of calcium ions polymerises to form ring-like structures within the membrane of target cells (Podack 1985, Henkart 1985, Tschopp and Conzelmann 1986, Young and Cohn 1986). These structures form transmembrane pores with an inner diameter of approximately 16 nm (Dennert and Podack 1985), causing functional disruption of the plasma membrane and leading to cell death.

In addition to Perforin, the cytoplasmic granules of CTLs contain a family of highly homologous serine esterase enzymes; these are termed granzymes A-H. These granzymes were recently isolated and characterised to some degree by Mason and Tschopp (1987), and exhibited a degree of structural homology as demonstrated by antigenic cross-reactivity, and N-terminal amino acid sequence homology. They also showed a spectrum of reactivity with the serine esterase affinity label diisopropylfluorophosphate; granzymes A and D reacted most strongly; B, E, F, G and H to a lesser extent; and C and F did not react at all.

Of these granzymes, A has been most extensively studied. It is a disulphide-linked dimer of molecular weight 60,000 and has been shown to catalyse the hydrolysis of several natural peptides and synthetic peptide derivatives witn trypsin-like specificity (Masson et al 1986a, 1986b and Pasternak et al 1986). It is expressed in CTLs or T helper cells with cytolytic activity, and although the precise role of proteolytic enzymes in cell mediated cytotoxicity is as yet unclear, it has been shown that alpha-1-antitrypsin and other protease inhibitors suppress CTL and NK cell induced target cell lysis (Chang and Eisen, 1980; Redelman and Hudig, 1980; Petty et al, 1984; Lavie et al 1985; and Goldfarb, l985).

Previously, all techniques have relied upon the use of cell lysates in either spectrophotometric assays or SDS polyacrylamide gels for activity levels and protein characterisation respectively of the enzyme and chromium 51 release cytotoxicity assays on whole cell preparations of variable cell types. These techniques require a large number of cells together with the use of specialist techniques and equipment. In addition, there is some doubt as to the maintenance of cell integrity in such assays.

We have now found a simple and rapid cytochemical staining method for detecting the presence and activity of the serine esterase enzyme system which may be used for example on whole cells in blood or bone marrow smears, paraffin embedded sections, cryostat tissue sections, and indeed where only limited number of cells are available, e.g. cerebrospinal fluid. This adapted staining method when used consecutively with a routine counterstain, or monoclonal antibodies, can readily identify a serine esterase positive cell.

According to one aspect of the present invention, there is provided a method of detecting serine

esterase in an animal cell or cell extract, which method comprises (a) fixing the sample which is to be tested, e.g. in formalin; and (b) contacting the fixed sample with a solution containing N-alpha benzyloxy carbonyl-L-lysine thiobenzyl ester and a chromogen (e.g. fast blue BB). Other fixative agents which may be used include dry acetone, ethanol, methanol and paraformaldehyde.

Preferably, cell preparations are assayed for serine esterase activity by incubation at 37°C tor 15 minutes using $2x10^{-4}$M N-alpha benzoxycarbonyl-L-lysine thiobenzyl ester (BLT) in 0.2M Tris-HCl buffer (pH 8.1) as enzyme substrate. This substrate has a low rate of non-enzymic hydrolysis under these conditions, and enzymic hydrolysis is detected by incorporating a chromogen at 0.2 mg $ml^{-1}$ within the system alongside the substrate. The chromogen binds to the reaction product, a benzyl mercaptan, allowing visualization of the sites of enzyme activity within the cells under test as intensely stained localized regions, once excess unbound chromogen has been washed off the cells. Should the reaction be allowed to proceed for significantly longer than the specified time, faint homogeneous nonspecific staining will appear as the chromogen reacts with other cellular components, as would be apparent on using chromogen alone.

Subsequently, the phenotype of individual cells may be determined by specific monoclonal antibody staining using alkali phosphatase/anti-alkali phosphatase (APAAP) techniques. It is important that staining be performed in this order, as APAAP staining will block serine esterase staining, although staining for serine esterase has no effect on APAAP staining.

Staining for serine esterase may also be performed on living cells by adding the BLT/chromogen mixture to the cell growth medium, when surface enzyme staining appears typically over a 10 minute period. Chromogen alone produces no staining at all in this case.

Staining for serine esterase is optimal at pH 8.1, although staining has been achieved at pH values from 7.0 to 8.5, independent of the nature of the buffer. However a slight decrease in the intensity of the staining at the extremes of this range is observed, which could lead to difficulties differentiating enzyme specific staining from non-specific staining under some circumstances. Hence a pH of 8.1 is preferred.

Optimal buffer strength for staining is 0.2 M although staining may be achieved in the range 0.01 M - (less intense staining) to 0.4 M (less precise localization of enzyme - more diffuse staining).

## Structure of BLT

The chemical structure of BLT is given below:

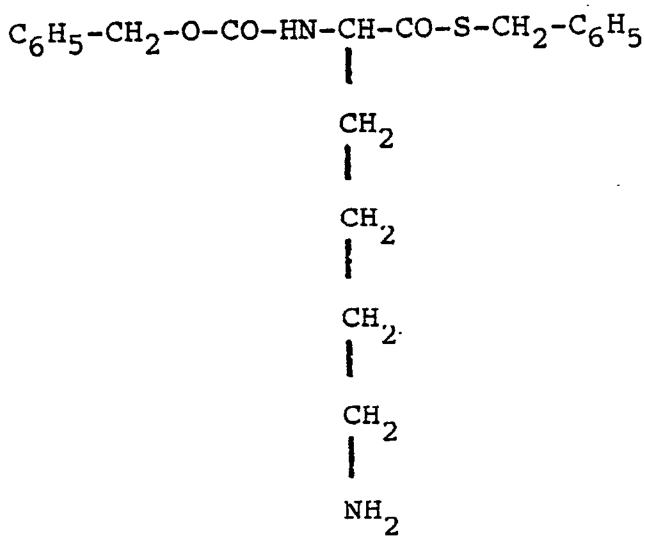

## Chromogens

The presently preferred chromogen is Fast Blue BB. We have tested this and other chromogens in the detection method of this invention, and the following table summarises the results:

| | Stain intensity |
|---|---|
| Fast Blue RR | + + |
| Fast Blue BB | + + + |
| Fast Blue B | + |
| Fast Violet | + |
| Fast Red TR | ± |
| Fast Garnet GBC | - |
| Fast Korinth V | ± |
| Ellman's Reagent | - |

We have found that the compound BLT is a substrate for some, but not all, serine esterases. It allows for recognition of certain esterases in leucocytes and some other cell types e.g. sperm cells, which have a membrane penetrating capacity as a functional requirement.

## Applications of enzyme staining

With regard to normal lymphoid tissue, some cells express serine esterase activity continually, and some not at all. In certain conditions, cells which did express serine esterase may show more staining, e.g. increased enzyme expression, while those which normally show no enzyme activity are induced to express the enzyme when activated. Eosinophils are normally serine esterase negative, as are macrophages and B lymphocytes, while basophils and neutrophils are serine esterase positive, and T lymphocytes are either positive or negative, depending on their sub-classification. Of these cell types, both macrophages and T lymphocytes become serine esterase positive when activated. Using this technique, it is possible to localize the cause of cellular activation by examining tissue sections, or identifying cells closely associated with activated cells, as well as demonstrating activation on a broad scale in blood or other bodily fluid smears.

Using the new technique we have identified serine esterase activity in helper T-cells in the peripheral circulation of patients with a number of auto-immune diseases, including Hashimoto's thyroiditis, insulin dependant diabetes, Sjogren's syndrome, systemic lupus erythromitosis and rheumatoid arthritis. In addition, it is possible to distinguish between various forms of leukaemia, particularly those in the myeloid series, since M1 and M5 types of the leukaemia do not demonstrate serine esterase acitivity, whereas types M2 and M4 carry the activity. Further evidence for serine esterase activity has been shown in patients undergoing rejection of renal transplants and this may be a simple method of detecting early marrow rejection. It also gives the physician the ability to distinguish between rejection and renal toxicity caused by immunosuppressants, e.g. cyclosporin.

Other possible applications for the method of serine esterase activity include the finding of decreased SE activity in patients with recurrent viral and bacterial infections; also it would be possible to monitor response to treatment in a number of malignant conditions, particularly leukaemias. We have found that treatment of hairy cell leukaemia with alpha interferon can be correlated with natural killer cell activity and the measurement of serine esterase on the natural killer cells.

According to a second aspect of the recent invention, there is provided a method of monitoring the treatment of auto-immune diseases, which comprises periodically assessing the serine esterase activity of cells from the tissues or regions of the body (e.g. lymphocytes) which are characteristically affected by the auto-immune disease. The invention also extends to monitoring treatment in other, non-auto-immune diseases which are characterised by serine esterase activity.

The following examples represent uses of these techniques in clinical situations. In each case, serine esterase activity was determined using smears of peripheral blood, bone marrow aspirates, cytocentrifuge preparations of mononuclear cells and cryostat tissue sections as appropriate. These were fixed in formalin vapour and immersed in the substrate (N-alpha benzyloxy carbonyl-L-lysine thiobenzyl ester (BLT) at $2 \times 10^{-4}$ molar concentration in 0.2 M Tris-HCl buffer at pH 8.1 together with the chromogen Fast Blue BB (0.2 mg per ml). Samples were immersed in the substrate solution for 15 minutes at 37°. After counter staining in haemotoxylin and mounting in aqueous mountant, slides were viewed under oil immersion.

## EXAMPLE 1

Lymphocytes have been located and identified (monoclonal/APAAP) infiltrating solid tumours. Benign tumours show no lymphocytic aggregation, whereas malignant tumours show infiltration by serine esterase positive macrophage (normally macrophages are negative), especially around any necrotic centers within the tumour. Thus macrophages are being stimulated to destroy tissue within malignant tumours, and this may be used in accordance with this invention as an indicator of response (or otherwise) to therapy, and possibly even as an early indicator of malignancy.

## EXAMPLE 2

In auto-immune disease, there is an increase in the number of lymphoid cells which stain for serine esterase and this change may be used diagnostically in accordance with the present invention.

a) Hashimoto's thyroiditis (autoimmunity to thyroglobulin) demonstrates an increase in the number of serine esterase-bearing lymphoid cells infiltrating the thyroid.

b) Insulin dependent diabetes shows an increase in serine esterase positive cells infiltrating the pancreas and in peripheral lymphoid cells.

c) In rheumatoid arthritis, an excess (compared with the norm) of peripheral lymphocytes show serine esterase staining.

## EXAMPLE 3

This Example relates to leukaemias.

a) Chronic lymphocytic leukaemia and myeloid leukaemia results in an increase in serine esterase expression in affected cells.

(b) No increase in enzyme expression is seen in acute B lymphoblast leukaemias, despite relative increases in the number of these cells, while in chronic cases, there is an increase in the number of T lymphocytes expressing enzyme activity, as well as the relative increase in the number of this type of lymphocyte.

Thus this could be used in accordance with this invention as an aid to differential diagnosis, and for monitoring therapy.

(c) Differential diagnosis of acute large blast cell leukaemias, e.g. myeloid vs granulocytic, is difficult on the basis of cell morphology, and is complex using existing cytochemical stains. However it is readily demonstrable with the technique according to the present invention, myeloid being serine esterase positive, and monocytic being negative.

## EXAMPLE 4

In monitoring kidney graft rejection, we have found that it is possible to differentiate between cyclosporin toxicity to the transplanted organ (a common occurence) and the onset of graft rejection. In both cases there is an increase in the number of T lymphocytes in the peripheral blood. In the former case, there is no increase in the relative number of serine esterase positive T lymphocytes, and the remedy appears to be to decrease cyclosporin dosage; while in the latter case, there is an increase in the relative number of serine esterase positive cells, and the remedy appears to be to increase the cyclosporin dosage.

In more general terms, an increase in the relative number of serine esterase positive T lymphocytes is indicative of the onset of graft rejection.

## EXAMPLE 5

In the field of male infertility, lack of serine esterase activity in immature spermatozoa is an indicator of incompetent cells, as the enzyme is important in the maturation process, and probably in fertilization as

well. Thus this could be used in accordance with this invention as part of tests carried out on semen samples to elucidate the cause of any infertility.

Agents that are capable of blocking the serine esterase system are expected to be of value in the treatment of auto-immune disorders such as those mentioned above, and also in diseases such as allergic encephalitis, Crohns disease and ulcerative colitis. Accordingly, in a further aspect, the present invention provides a method for the treatment of auto-immune diseases, which comprises administering a therapeutic amount of a pharmaceutical composition containing, in active form, a compound or composition capable of blocking serine esterase activity.

The invention also provides a pharmaceutical composition containing an effective amount of a compound or composition capable of blocking serine esterase activity.

## Claims

1. A method of detecting serine esterase in an animal cell or cell extract, which method comprises (a) fixing the sample which is to be tested, e.g. in formalin; and (b) contacting the fixed sample with a solution containing N-alpha benzyloxy carbonyl-L-lysine thiobenzyl ester and a chromogen (e.g. fast blue BB).

2. A method of monitoring the treatment of auto-immune diseases, which comprises periodically assessing the serine esterase activity of cells from the tissues or regions of the body (e.g. lymphocytes) which are characteristically affected by the auto-immune disease.

3. A method for the treatment of auto-immune diseases, which comprises administering a therapeutic amount of a pharmaceutical composition containing, in active form, a compound or composition capable of blocking serine esterase activity.

4. A pharmaceutical composition containing an effective amount of a compound or composition capable of blocking serine esterase activity.

5 A method of differentiating between malignant and benign tumours, in which tissue samples of the tumour to be analysed is subjected to the method of claim 1.

6. A method of monitoring the treatment of malignant diseases, which comprises periodically assessing the serine esterase activity of cells from the tissues or regions of the body (e.g. lymphocytes) which are characteristically affected by the disease.

7. A method of monitoring the onset of graft rejection in a tissue graft, in which tissue samples of the grafted tissue are subjected to the method of claim 1.

8. A method of testing the competency of spermatozoa, in which cell extracts from the spermatozoa to be tested are subjected to the method of claim 1.

9. A method of detecting serine esterase in an animal cell or cell extract, which method comprises (a) fixing the sample which is to be tested; and (b) contacting the fixed sample with a buffered solution containing N-alpha benzyloxy carbonyl-L-lysine thiobenzyl ester and the chromogen Fast Blue BB at a pH in the range 7.0 to 8.5.

10. A method as claimed in claim 9, wherein step (b) is carried out at a temperature of about 37$^\circ$C for about 15 minutes.

11. A method as claimed in claim 9, wherein step (b) is carried out with the BTL ester and chromogen in 0.2M Tris-HCl buffer solution.

12. A method as claimed in claim 9, wherein step (b) is carried out with the BTL ester and chromogen at respective concentrations of 2 x $10^{-4}$M and 0.2mg/ml.

13. Use of N-alpha benzyloxy carbonyl-L-lysine thiobenzyl ester and the chromogen Fast Blue BB as an indicator of Serine Esterase activity.